(19) Europäisches Patentamt
European Patent Office
Office européen des brevets

(11) Publication number: 0 007 985
B1

(12) # EUROPEAN PATENT SPECIFICATION

(45) Date of publication of patent specification: 01.09.82

(21) Application number: 79101778.3

(22) Date of filing: 05.06.79

(51) Int. Cl.[3]: C 07 C 50/32, C 07 C 69/00, C 07 C 46/00, A 61 K 31/12, A 61 K 31/215

(54) 6-Chloro-2,3-dihydroxy-1,4-naphthoquinone and its 2,3-diesters, and their preparation.

(30) Priority: 05.06.78 US 912697

(43) Date of publication of application: 20.02.80 Bulletin 80/4

(45) Publication of the grant of the patent: 01.09.82 Bulletin 82/35

(84) Designated Contracting States: AT BE CH DE FR GB IT LU NL SE

(56) References cited:
Chemische Berichte, Vol. 80, Nr. 5, 1947, Berlin, Heidelberg F. WEYGAND et al. "Synthesen von 2.3-Dioxynaphthochinonen aus substituierten o-Phthalaldehyden und Glyoxal" pages 391 to 401

(73) Proprietor: SYNTEX (U.S.A.) INC.
3401 Hillview Avenue
Palo Alto, California 94304 (US)

(72) Inventor: Jones, Gordon H.
21550 Edward Way
Cupertino, California 95014 (US)
Inventor: Young, John M.
300 Summit Drive
Redwood City, California 94062 (US)

(74) Representative: Weinhold, Peter, Dr. et al,
Patentanwälte Dipl.-Ing. P. Wirth Dr. V. Schmied-Kowarzik Dipl.-Ing. G. Dannenberg Dr. P. Weinhold Dr. D. Gudel Dipl.-Ing. S. Schubert
Siegfriedstr. 8
D-8000 München 40 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 007 985 B1

Courier Press, Leamington Spa, England.

6-Chloro-2,3-dihydroxy-1,4-naphthoquinone and its 2,3-diesters, and their preparation

This invention relates to novel naphthoquinones which are of pharmaceutical use in treating psoriasis in a mammal.

Psoriasis is a skin disease characterized in part by excessive proliferation of cells of the epidermis which remain strongly adherent and build up into a scaley plaque typical of the disease. Currently available therapies, which are not curative, depend on the control of epidermal cell proliferation through the use of hormonal agents, such as corticosteroids, or through the use of compounds related to cancer chemotheraphy such as hydroxyurea, methotrexate, and the nitrogen mustards.

While the above agents are effective to a certain extent, they cause numerous severe undesirable side effects both locally and systemically.

Certain naphthoquinones which are chemically related to the compound of this invention are known. For example, the compound 6-bromo-2,3-dihydroxy-1,4-naphthoquinone has been reported by Weygand, German 859,008. While this compound is recognized as a valuable intermediate for the preparation of dyestuffs, no useful biological activity has been ascribed to it. Specifically, any suggestion that this compound might have anti-psoriatic activity has not been advanced.

Related naphthoquinones having antifungal and antibacterial activity can be found in U.S. letters patent 3,914,264 and West German Offenlegungsschrift 2,135,712, 2,456,655 and 2,520,739. However, none of these disclosures have recognized the use of these or related naphthoquinone compositions as anti-psoriatic agents.

It is also suggested that certain related compounds have antimalarial activity, e.g. 6-chloro-2-hydroxy-1,4-naphthoquinone, 7-chloro-2-hydroxy-1,4-naphthoquinone and 6-chloro-1,2,4-triacetoxy-naphthoquinone. See L. F. Fieser and R. H. Brown, J. Am. Chem. Soc. *71*, 3615—17 (1949).

It has now been discovered that the novel compound 6-chloro-2,3-dihydroxy-1,4-naphthoquinone and its 2,3-diesters are effective in preventing epidermal cell proliferation when topically applied to a mammal. More specifically, it is effective as an anti-psoriatic agent in humans.

In its broadest aspect, the present invention is the compound 6-chloro-2,3-dihydroxy-1,4-naphthoquinone and its 2,3-diesters represented by Formula (I), below.

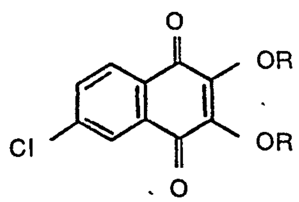

(I)

wherein R is hydrogen or $R^1C(O)$— where $R^1$ is an alkyl of 1—5 carbon atoms.

Another aspect provides a pharmaceutical composition comprising a compound of the invention together with at least one pharmaceutically acceptable carrier.

Still another aspect of this invention is a process for preparing the compound of Formula (I) where R is hydrogen by reacting 4-chloro-1,2-phthalic aldehyde with glyoxal in the presence of an aqueous base or by treating 6-chloro-2-hydroxy-1,4-naphthoquione, 7-chloro-2-hydroxy-1,4-naphthoquinone or mixtures thereof with an oxidizing agent.

The final aspect of this invention is the preparation of a compound of Formula (I), wherein R is $R^1C(O)$— and $R^1$ is alkyl of 1—5 carbon atoms, which process comprises esterifying a compound of Formula (I) wherein R is H.

Formulation and Administration

The compounds according to the present invention are formulated for administration in any convenient way by analogy with other topical compositions. These compositions may be presented for use in any conventional manner with the aid of any of a wide variety of pharmaceutical carriers or vehicles.

Generally, the psoriatic manifestation in mammals, particularly humans, is combatted by contacting the afflicted area with a therapeutically effective amount of the naphthoquinone of this invention, that is, an amount which reduces the proliferation of epidermal cells (an anti-psoriatic effect). Preferably, the naphthoquinone is first formulated to prepare a suitable pharmaceutical formulation, as discussed hereinafter, which is then placed in contact with an afflicted area. An effective amount of the napthoquinone will depend upon the particular condition and the mammal receiving the treatment and will vary between 0.001% to 10% by weight of the pharmaceutical composition and preferably will be between 0.01% and 1% by weight of the formulation. Using these levels in the formulation, a therapeutically effective and non-side effect producing amount, i.e. enough to effect an antipsoriatic response, but not enough to adversely effect the recipient, is applied to the afflicted area(s).

The naphthoquinone of this invention may be formulated with suitable pharmaceutical vehicles known in the art to form particularly effective topical compositions. As disclosed above, an effective

amount of the naphthoquinone is about 0.001%w to about 10%w of the total formulated composition. The rest of the formulated composition will be about 90%w to about 99.999%w of a suitable excipient which may include a pharmaceutically acceptable solvent and other pharmaceutically acceptable additives to form a topically effective pharmaceutical formulation.

A pharmaceutically acceptable solvent is one which is substantially non-toxic and non-irritating under the conditions used and may be readily formulated into any of the classical drug formulations such as powders, creams, ointments, lotions, gels, foams, aerosols, solutions and the like. Particularly suitable solvents include water, ethanol, acetone, glycerine, propylene carbonate, dimethyl-sulfoxide (DMSO), and glycols such as 1,2-propylene diol, i.e., propylene glycol, 1,3-propylene diol, polyethylene glycol having a molecular weight of from 100 to 10,000, dipropylene glycol, etc. and mixtures of the aforementioned solvents with each other.

A topical cream may be prepared as a semi-solid emulsion of oil in water or water in oil. A cream base formulation by definition is an emulsion, which is a two-phase system with one liquid (for example fats or oils) being dispersed as small globules in another substance (e.g., a glycol-water solvent phase) which may be employed as the primary solvent for the napthoquinones therein. The cream formulation may contain fatty alcohols, surfactants, mineral oil or petrolatum and other typical pharmaceutical adjuvants such as anti-oxidants, antiseptics, or compatible adjuvants. A typical cream base formulation is as follows:

| | |
|---|---|
| Water/glycol mixture (15% or more glycol) | 50—99 parts by weight |
| Fatty Alcohol | 1—20 |
| Non-ionic Surfactant | 0—10 |
| Mineral Oil | 0—10 |
| Typical Pharmaceutical Adjuvants | 0— 5 |
| Active Ingredients | 0.001—10 |

The fatty alcohol, non-ionic surfactants, and other adjuvants are discussed in U.S. 3,934,013 to Poulsen which is incorporated herein by reference.

The naphthoquinones of this invention may also be formulated as topical ointments. A "classical" ointment is a semisolid anhydrous composition which may contain mineral oil, white petrolatum, suitable solvent such as a glycol and may include propylene carbonate and other pharmaceutically suitable additives such as surfactants, for example Span and Tween, or wool fat (lanolin), along with stabilizers such as antioxidants and other adjuvants as mentioned before. Following is an example of a typical "classical" ointment base:

| | | |
|---|---|---|
| White Petrolatum | 40—94 | parts by weight |
| Mineral Oil | 5—20 | |
| Glycol Solvent | 1—15 | |
| Surfactant | 0—10 | |
| Stabilizer | 0—10 | |
| Active Ingredients | 0.001—10.0 | |

Other suitable ointment base formulations which employ propylene carbonate are described in U.S. patent 4,017,615 issued April 12, 1977 by Shastri et al entitled "Propylene Carbonate Ointment Vehicle" and U.S. 3,924,004 issued December 2, 1975 by Chang et al entitled "Fatty Alcohol-Propylene Carbonate-Glycol Solvent Cream Vehicle". As much of those applications as is pertinent is incorporated herein by reference. Following is a typical ointment base formulation containing propylene carbonate:

| | |
|---|---|
| Active Ingredients | 0.001—10.0 parts by weight |
| Propylene Carbonate | 1—10 |
| Solvent | 1—10 |
| Surfactant | 0—10 |
| White Petrolatum | 70—97 |

Suitable solvents, surfactants, stabilizers, etc. are discussed in U.S. 3,934,013 and such are incorporated herein by reference.

A suitable topical "non-classical" anhydrous, water washable "ointment type" base is described in U.S. Patent No. 3,592,930 to Katz and Neiman, and that patent is incorporated herein by reference. A representative composition of this invention utilizing such base is as follows:

| | | |
|---|---|---|
| Glycol Solvent | 40—35 | parts by weight |
| Fatty Alcohol | 15—45 | |
| Compatible Plasticizer | 0—15 | |
| Compatible Coupling Agent | 0—15 | |
| Penetrant | 0—20 | |
| Active Ingredients | 0.001—10.0 | |

*Process for Preparation*

The naphthoquinone of formula (I) wherein R is H is prepared following, in principle, the process disclosed in the aforesaid Weygand patent and illustrated by the following Reaction Sequence:

REACTION SEQUENCE 1

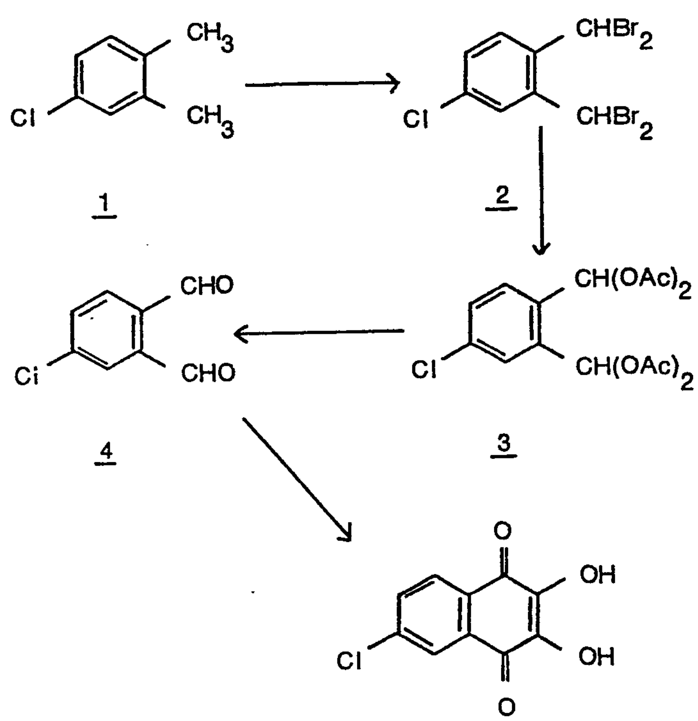

1 2 4 3 5

Initially, the methyl groups of the chloro substituted ortho-xylene (compounds *1*) are directly halogenated. Disubstitution of halo on each of these groups is found to be most facile when bromo is

the desired halo substituent. The reaction occurs readily using N-bromosuccinimide as the brominating agent. See for example, Djerassi, Chem. Revs., *43*, 271 (1948). Compound *2*, the 4-chloro-alpha, alpha, beta, beta-tetrabromo-ortho-xylene, is next dehalogenated in a manner known for the hydrolysis of gem-dihalides in the presence of a suitable catalyst. The catalysts include, for example, calcium carbonate, sulfuric acid, morpholine and various silver salts. Typically a mixture of acetic acid, compound *2* and silver acetate is heated, resulting in dehalogenating compound *2* to compound *3* which is readily converted to compound *4* typically with base such as sodium bicarbonate. The 4-chloro-1,2-phthalic aldehyde intermediate (*4*) is next converted according to the process of this invention to the 6-chloro-2,3-dihydroxy-1,4-naphthoquinone compound *5* by reaction with glyoxal (OHCCHO). This reaction is reported in some detail in the aforesaid German Patentschrift No. 859,008, which is incorporated herein by reference. Generally the reaction is carried out in an aqueous base at a pH of between 8 and 12 and at temperatures of 10° to 80°C. The bases which are employed include alkali metal carbonates, bicarbonates and hydroxides, tertiary amines and the like.

The compound of Formula (I) wherein R is H is also prepared by oxidizing 6-chloro-2-hydroxy-1,4-naphthoquinone or 7-chloro-2-hydroxy-1,4-naphthoquinone according to Reaction Sequence 2.

REACTION SEQUENCE 2

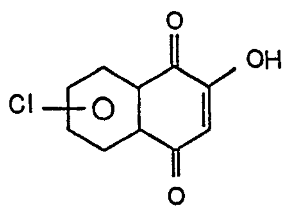

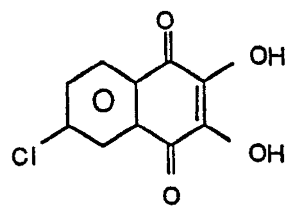

Both precursors are known compounds. See, for example, J. Am. Chem. Soc. *71*, 3615—17 (1949) and J. Am. Chem. Soc. *75*, 2910—15 (1953). The oxidation is suitably carried out by employing methods known in the art such as basic hydrogen peroxide, peracids, e.g., peracetic acid, m, chlorparbenzoic acid, potassium ferricyanid aqueous in alkaline solution, and the like, preferably basic hydrogen peroxide.

The compounds of Formula (I) wherein R is $R^1C(O)$— are prepared by esterifying the 2,3-dihydroxy compound using methods known in the art. For example, with acyl anhydride or chloride in pyridine solution or in an inert solvent such as tetrahydrofuran containing a tertiary base such as pyridine or triethylamine, with acyl anhydride in the presence of an acid catalyst such as zinc chloride, sulfuric acid or 70% perchloric acid.

For Preparations 1 and 2 and Examples 1 and 2, reference should be made to the Reaction Sequence 1.

*Preparation 1*

4-Chloro-$\alpha,\alpha,\beta,\beta$-tetrabromo-o-xylene

A mixture of 4-chloro-o-xylene (*1*: 14.0 g, 0.1 mol) and freshly recrystallized N-bromosuccinimide (80 g, 0.45 mol) in carbon tetrachloride (1 L) is irradiated for 2 1/2 hours (hr) with a 250 watt tungsten infrared lamp positioned so that the solvent refluxed gently. The solid material is then removed by filtration, the filtrate is concentrated in vacuo and the crystalline residue is recrystallized from isopropanol giving 34 g (75%) of 4-chloro-$\alpha,\alpha,\beta,\beta$-tetrabromo-o-xylene (*2*), melting point (mp) 108—111°C [Bull. Soc. Chim., 2966 (1966), mp 114°].

*Preparation 2*

4-Chloro-1,2-phthalicaldehyde

A mixture of *2* (22.8 g, 0.05 mol) silver acetate (36,6 g, 0.22 mol) and glacial acetic acid (500 ml) is heated under reflux for 1—1/2 hr. The silver salts are filtered off, washed with chloroform and the combined filtrates concentrated to dryness in vacuo. The last traces of acetic acid are removed from the residue by evaporation with toluene. The resultant semicrystalline material *3* is dissolved in dioxane (500 ml) and a solution of sodium carbonate (53 g) in water (500 ml) is added. This mixture is shaken vigorously at 22° for 20 minutes, saturated with sodium chloride and then extracted with ethyl acetate (2 x 500 ml). The combined organic extracts are washed with brine (2 x 400 ml), dried with $MgSO_4$ and concentrated in vacuo giving 6.0 g (72%) of almost pure 4-chloro-1,2-phthalic aldehyde. Sublimation at 75° and 0.05 mm Hg affords 5.4 g of *4*, with mp 72—76°.

Example 1

6-Chloro-2,3-dihydroxy-1,4-naphthoquinone

A solution of sodium carbonate (12.3 g) in water (290 ml) is added at room temperature to a vigorously stirred solution of 4-chloro-1,2-phthalic aldehyde (4.88 g, 29 mmol) in an oxygen

atmosphere followed immediately by the addition, over a five minute period, of a solution of glyoxal bisulfite (16.4 g, 58 mmol) and potassium cyanide (1.88 g, 29 mmol) in water (290 ml). The reaction mixture is stirred in the oxygen atmosphere for a further 5 minutes. It is then treated with 6 N hydrochloric acid (50 ml), cooled in an ice-salt bath and further diluted with water (500 ml). The crystalline product is collected by filtration, washed with water, dried and recrystallized from water: methanol (2:1) giving 3.56 g (55%) of 6-chloro-2,3-dihydroxy-1,4-naphthoquinone, mp 228—229°.

*Anal.* Calcd. for $C_{10}H_5ClO_4$ (224.60): C, 53.48; H, 2.24.
Found: C, 53.50; H, 2.30.

Example 2

Since known effective anti-psoriasis agents inhibit epidermal cell proliferation, an animal assay using young male rats has been developed wherein the test agent in an appropriate carrier is applied to the skin of one flank of the test animal while the carrier alone is applied to the other flank, i.e., the control. At a suitable time interval after one or more applications of the composition, the skin is removed from the animal and skin plugs taken from both the treated and the control areas are incubated *in vitro* with tritium labelled thymidine (a precursor of DNA synthesis). A comparative measure of the amount of radio-labelled thymidine incorporated into the DNA of plugs from the treated and control flanks gives a measure of the degree of inhibition of DNA synthesis and hence epidermal cell proliferation which has occurred in response to the treatment.

Compositions illustrative of the compositions of the present invention are tested in the rat in the method disclosed above. The tests are specifically carried out as follows:

Male albino rats, 21—22 days old are shaved on the back and both flanks with electric clippers twenty-four hours prior to testing. 6-Chloro-2,3-dihydroxy-1,4-naphthoquinone was dissolved in a mixture of ethanol (EtOH), acetone, dimethyl sulfoxide (DMSO) or mixture of DMSO and EtOH. Solutions (0.10 ml) containing the test agent, are supplied to the right flank of the animals and the carrier alone (0.10 ml) is applied to the left flank. The area covered is approximately 10 sq. cm. Applications are made at 9:00 a.m. and 4:00 p.m. on days 1, 2 and 3. On day 4 the animals are sacrificed by carbon dioxide asphyxiation, and the full thickness skin from the back and both flanks are removed. Subcutaneous fat and muscle are scraped away and plugs consisting of epidermis and dermis, 6 mm. in diameter are punched out, five from the control flank and five from the treated flank. These are incubated at 37° individually floating on 3 ml of Dulbecco's Modified Eagle Medium containing tritiated thymidine (10 microcuries/milliliter). After two hours incubation the plugs are frozen, thawed, blotted dry, rinsed 3 times in 30% aqueous acetic acid and once in absolute ethanol to remove unincorporated tritiated thymidine. The washed plugs are digested in NCS solubilizer for liquid scintillation counting in order to determine the amount of tritiated thymidine incorporated into the DNA. The inhibitory effect of the treatment is determined by comparing the amount of radioactivity incorporated into plugs from the treated side with that incorporated into plugs from the control flank. For each test at least two animals are used. The data are recorded for each animal as the ratio of mean radioactivity in the treated side to the mean radioactivity of the control side (X100).

The amount of tritiated thymidine incorporated is primarily due to epidermal cells, and hence the amount of radioactivity in these tests is directly related to the number of cells (epidermal) engaged in proliferation (DNA synthesis) at the moment of sacrifice. As noted earlier, epidermal cell hyper-proliferation is symptomatic of psoriasis. The compound showed inhibitory activity against epidermal cell proliferation and as a result is an anti-psoriatic agent.

| % Concentration solvent (ratios) | Effectiveness[1] |
|---|---|
| 2% DMSO/EtOh (1/1) | 69, 56 |
| 2% DMSO/EtOH/Acetone (1/4.5/4.5) | 85, 82 |

[1] $\frac{\text{Mean Radioactivity treated side}}{\text{Mean Radioactivity Control}} \times 100$

Example 3

24 Swiss-Webster mice (Simonsen), each weighing about 25 grams, were divided into 4 groups of 6 mice each. The mice in each of 3 of the groups were injected subcutaneously with a solution of 6-chloro-2,3-dihydroxy-1,4-naphthoquinone in carbomethoxycellulose. The dosage in each of the 3 groups was 10 mg/kg, 50 mg/kg and 250 mg/kg, respectively. This was about .25 mg/mouse, 1.250 mg/mouse and 6.25 mg/mouse in each of the respective groups. The control group was injected subcutaneously with carbomethoxy-cellulose.

The test material was given as indicated on day one only and the mice were observed daily for mortality for 14 days. No deaths occurred in any of the groups. The $LD_{50}$ is, therefore greater than 250 mg/kg.

Example 4

A solution of 1.2 gm 6-chloro-2-hydroxy-1,4-naphthoquinone (or 7-chloro or mixture thereof) and 0.48 g sodium bicarbonate in 58 ml of water is cooled to 5°C and 2.3 ml of 30% hydrogen peroxide is added in one portion. The mixture is then stirred at 22°C for 16 hours where the red crystalline product (0.24 g) is removed by filtration and crystallized from water:methanol (2:1) giving the 6-chloro-2,3-dihydroxy-1,4-naphthoquinone, m.p. 228—229°C.

Example 5

Concentrated sulfuric acid (0.1 ml) is added to a suspension of 1 g of 6-chloro-2,3-dihydroxy-1,4-naphthoquinone in 5 ml of acetic anhydride and the mixture is gently shaken at 22°C until a clear, pale yellow solution is obtained (less than 5 minutes). After a further 5 minutes at 22°C the solution is poured into 250 ml of saturated sodium bicarbonate solution and the yellow crystalline material is collected by filtration, washed well with water and dried in vacuo giving 1.05 g of the 2,3-diacetoxy 6-chloro-1,4-naphthoquinone, m.p. 109—110°C.

Example 6

Concentrated sulfuric acid (0.1 ml) is added to a suspension of 1 g of 6-chloro-2,3-dihydroxy-1,4-naphthoquinone in 5 ml of caproic anhydride and the mixture is gently shaken at 22°C until a clear, pale yellow solution is obtained (less than 5 minutes). After a further 10 minutes at 22°C the solution is poured into 250 ml of saturated sodium bicarbonate solution. The product (2,3-dicaproxyloxy-6-chloro-1,4-napthoquinone) is then isolated from the resultant organic layer.

**Claims**

1. A compound selected from those represented by the formula

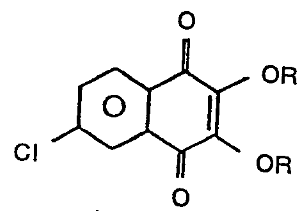

wherein R is hydrogen or $R^1C(O)$— and $R^1$ is alkyl or one to five carbon atoms.

2. A pharmaceutical composition which comprises a therapeutically effective amount of a compound selected from those represented by the formula

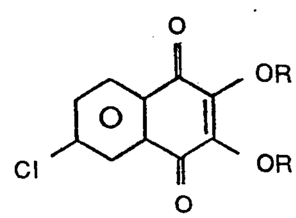

wherein R is hydrogen or $R^1C(O)$— and $R^1$ is alkyl of one to five carbon atoms in combination with a pharmaceutically acceptable, non-toxic carrier.

3. A process for preparing a compound selected from those represented by the formula

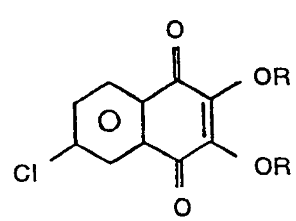

wherein R is hydrogen which comprises

(a) reactively contacting 4-chloro-1,2-phthalic aldehyde with glyoxal or

(b) oxidizing 6-chloro-2-hydroxy-1,4-naphthoquinone, 7-chloro-2-hydroxy-1,4-naphthoquinone or

mixtures thereof from 0°C to the boiling temperature of the reaction medium.

4. A process for preparing a compound selected from those represented by the formula

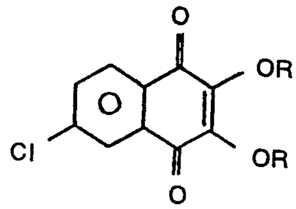

wherein R is $R^1C(O)$— and $R^1$ is alkyl of one to five carbon atoms which process comprises diesterifying a compound of the formula

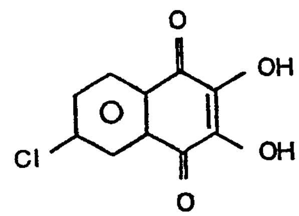

at a temperature of −10°C to 50°C.

**Patentansprüche**

1. Eine Verbindung, ausgewählt aus Verbindungen der Formel:

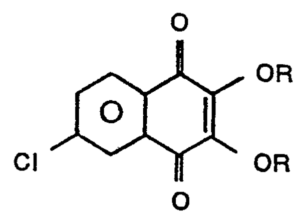

in welcher R für Wasserstoff oder $R^1C(O)$— steht und $R^1$ Alkyl mit 1 bis 5 Kohlenstoffatomen bedeutet.

2. Pharmazeutisches Präparat, enthaltend eine therapeutische Menge einer Verbindung, ausgewählt aus Verbindungen der Formel:

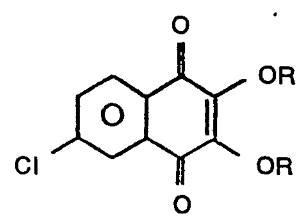

in welcher R für Wasserstoff oder $R^1C(O)$— steht und $R^1$ Alkyl mit 1 bis 5 Kohlenstoffatomen bedeutet, in Kombination mit einem pharmazeutisch annehmbaren, nicht toxischen Träger.

3. Verfahren zur Herstellung einer Verbindung, ausgewählt aus Verbindungen der Formel:

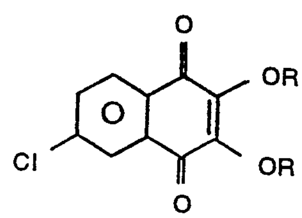

in welcher R für Wasserstoff steht, dadurch gekennzeichnet, daß
(a) 4-Chlor-1,2-phthalaldehyd mit Glyoxal reaktiv in Berührung gebracht wird oder
(b) 6-Chlor-2-hydroxy-1,4-naphthochinon, 7-Chlor-2-hydroxy-1,4-naphthochinon oder Mischungen

derselben zwischen 0°C und der Siedetemperatur der Reaktionsmischung oxidiert werden.

4. Verfahren zur Herstellung einer Verbindung, ausgewählt aus Verbindungen der Formel:

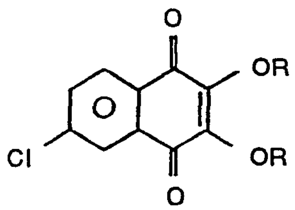

in welcher R für $R^1C(O)$— steht und $R^1$ Alkyl mit 1 bis 5 Kohlenstoffatomen bedeutet, dadurch gekennzeichnet, daß man eine Verbindung der Formel

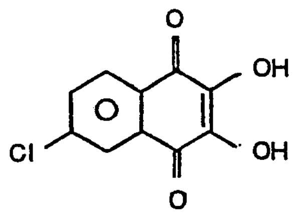

bei einer Temperatur von −10°C bis 50°C diesterifiziert.

## Revendications

1. Composé choisi parmi ceux qui sont représentés par la formule:

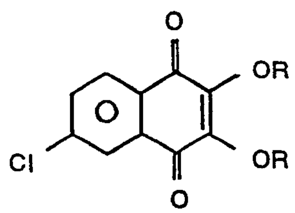

dans laquelle R est l'hydrogène ou un groupe $R^1C(O)$— et $R^1$ est un radical alkyle ayant 1 à 5 atomes de carbone.

2. Composition pharmaceutique, qui comprend une quantité thérapeutiquement efficace d'un composé choisi parmi ceux qui sont représentés par la formule:

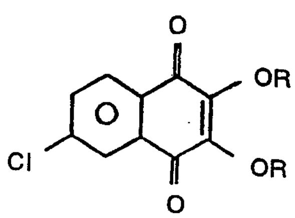

dans laquelle R est l'hydrogène ou un groupe $R^1C(O)$— et $R^1$ est un radical alkyle ayant 1 à 5 atomes de carbone, en association avec un support non toxique pharmaceutiquement acceptable.

3. Procédé de préparation d'un composé choisi parmi ceux qui répondent à la formule:

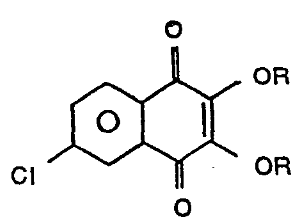

dans laquelle R est l'hydrogène, qui consiste:
(a) à faire entrer en contact réactionnel l'aldéhyde 4-chloro-1,2-phtalique avec le glyoxal ou
(b) à oxyder la 6-chloro-2-hydroxy-1,4-naphtoquinone, la 7-chloro-2-hydroxy-1,4-naphtoquinone ou

leurs mélanges à une température allant de 0°C à la température d'ébullition du milieu de réaction.

4. Procédé de préparation d'un composé choisi parmi ceux qui répondent à la formule:

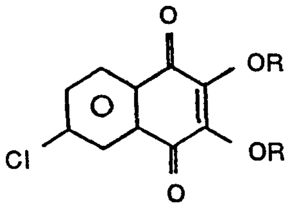

dans laquelle R est un groupe $R^1C(O)$— où $R^1$ est un radical alkyle ayant 1 à 5 atomes de carbone, procédé qui consiste à diestérifier un composé de formule:

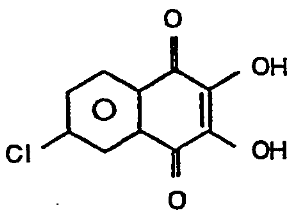

à une température de —10 à 50°C.